# EUROPEAN PATENT SPECIFICATION

(11) **EP 1 537 872 B1**
(45) Date of publication and mention of the grant of the patent: **09.05.2018**
(21) Application number: 03733607.0
(22) Date of filing: 13.06.2003
(51) Int. Cl.: A61K 35/76

(54) **IMMUNOSTIMULATOR HAVING ANTINEOPLASTIC ACTION AND METHOD FOR PRODUCING SAID IMMUNOSTIMULATOR**
IMMUNSTIMULATOR MIT ANTINEOPLASTISCHER WIRKUNG UND VERFAHREN ZUR HERSTELLUNG DES IMMUNOSTIMULATORS
IMMUNOSTIMULATEUR PRESENTANT UNE ACTIVITE ANTICANCEREUSE ET PROCEDE DE PREPARATION CORRESPONDANT

(30) Priority: 14.06.2002 LV 020108
(43) Date of publication of application: 08.06.2005
(73) Proprietor: Ditesan Ltd., Riga 1050 (LV)
(72) Inventor: MUCENIECE, Aina, 1058 RIGA (LV)
(74) Representative: Madgwick, Paul Roland
(86) International application number: PCT/LV2003/000006
(87) International publication number: WO 2003/105875

(56) References cited:
- WO-A1-01/37866
- US-A- 4 315 914
- FERDAT A.K. ET AL.: 'Mekhanizm immunomoduliasty b potivoopukholevom deistvy enterobiruca, ECHO-7' EKSPERIMENTALNAYA vol. 11, no. 5, 1999, pages 43 - 48, XP008054566
- '10.3 GENDON, genetika virusof cheloveka i zhibotykh' NAUKA 1967, MOSCOW, pages 216 - 217, 228-230, XP008097756
- 'Rukovodstvo po laboratornoi diagnostike virusnyx i rikketsioznyx boleznei' MEDISTINA 1965, MOSCOW, pages 330 - 331, XP008097757
- FERDATS A ET AL: "P17 Adjuvant immunotherapy with echovirus vaccine for malignant melanoma", MELANOMA RESEARCH, LIPPINCOTT WILLIAMS & WILKINS, US, vol. 9, no. 3, 1 January 1999 (1999-01-01) , pages 329-330, XP009156677, ISSN: 0960-8931
- SINKOVICS J G ET AL: "NEWCASTLE DISEASE VIRUS (NDV): BRIEF HISTORY OF ITS ONCOLYTIC STRAINS", JOURNAL OF CLINICAL VIROLOGY, ELSEVIER, AMSTERDAM, NL, vol. 16, no. 1, 1 January 2000 (2000-01-01), pages 1-15, XP000982846, ISSN: 1386-6532, DOI: 10.1016/S1386-6532(99)00072-4
- Pamela M. Kelly ET AL: "Replication of encephalomyocarditis virus in various mammalian cell types", Journal of Medical Virology, vol. 11, no. 3, 1 January 1983 (1983-01-01), pages 257-264, XP055150638, ISSN: 0146-6615, DOI: 10.1002/jmv.1890110309
- Tuck Weng Kok ET AL: "Comparison of rhabdomyosarcoma, buffalo green monkey kidney epithelial, A549 (human lung epithelial) cells and human embryonic lung fibroblasts for isolation of enteroviruses from clinical samples", Journal of Clinical Virology, vol. 11, no. 1, 1 July 1998 (1998-07-01), pages 61-65, XP055150640, ISSN: 1386-6532, DOI: 10.1016/S0928-0197(98)00026-9

## Description

The proposed invention relates to the field of medicine, to the methods of producing preparations for active immunotherapy of malignant tumors, e.g., malignant melanoma.

At present is known the immunostimulator consisting of the mixture of tumor antigens and the source of Mn⁺⁺ (manganese ions) (UK patent No.1592496, 24.11.1977 and French patent No. 2423223, 20.05.1979 /Int. Class. A61K39/00/). Such immunostimulator proved to be effective at the early stages of disease or in case of secondary treatment with small amount of cells remaining after the first treatment.

Nevertheless, if administration should be performed into considerable tumor, the injection could appear worthless or even makes things worse due to increasement of number of circulating tumor antigens, in its turn blocking the immune cells.

The possibilities of preparing the specific vaccines from the individual tumors are limited by the time and the amount of the material available.

Also are known immunostimulating adjuvants containing the primary antigen from killed strain of B.Pertussis NRRL-11,232 coupled by diisocyanate with the second antigen from live cells of adenocarcinoma (US patent No.4285930, 13.11.1979/ Int.Class. A61K39/12/).

The application of mentioned stimulators increased the efficiency of complex therapy. At the same time application of such preparations is accompanied by side effects, both local and general, e.g., inflammation granulomas, ulcers, lymphadenopathia, phenomenons of hypersensitivity, etc.

From the known stimulators the most similar to the proposed immunostimulator of cellular immunity with antitumoral effect is the one that contains the attenuated viral culture of Vaccinia virus, strain AS ATTC No. VR 2010 (US patent No. 4315914, 04.04.1980 /Int.Class. A61K45/05/).
Immunostimulator is produced by isolation of non-pathogenic Vaccinia virus, passaging it in monolayer culture of rat kidney cells and propagation of it in monolayer culture of chick embryo.

This immunostimulator is represented by the biggest, structurally and antigenically complex DNA -genomic virus, expressing itself as the polyclonal stimulator of the immune system, affecting mostly the cellular immunity. In case of lasting parenteral administration of it various side-effects could appear, e.g., reactions of hypersitivity which are typical for bacterial nonspecific immunostimulators as well.

These side-effects reduce the effectiveness of application of the preparation aimed to suppress the development of tumor appearing after the operation.

In many cases these side-effects make the application of this preparation impossible in general.

Coxsackie A.group viruses have been shown to be active against malignant cells *in vitro* and *in vivo* and Echovirus type 7 *in vitro* (International publication WO 01/37866 31.5.2001 /Int.Class. A61K39/12/), but the preparation of Echovirus is not discussed. Even if the viruses belong to the same family, it is not automatic result from this that they are all equally oncotropic and oncolytic.

Ferdats et al. in Melanoma Research 9: 329-330 (1999) indicate that an echovirus vaccine has been developed and tested, but gives no technical details on development and manufacturing such vaccine product using type E7.

Sinkovics et al. in Journal of Clinical Virology 16: 1-15 (2000) discuss mainly the oncolytic properties of Newcastle disease virus (NDV) and mentions briefly an echovirus strain used as vaccine to prevent relapses of melanoma, but no exact steps of preparing the vaccine are given.

Kelly et al. in Journal of Medical Virology 11: 257-264 (1983) studies replication of encephalomyocarditis virus, belonging to the family Picornaviridae, *in vitro* in various mammalian cell types, including human fibroblasts and melanoma cells.

The object of this invention is to present a method for preparation of an immunostimulator with antitumoral effect *in vivo* comprising nonpathogenic virus strain in order to increase the efficiency of suppression of malignant growth after operation in case of long-lasting administration.
The object of invention is achieved due to the fact, that in the method of producing of the immunostimulator the nonpathogenic virus strain is obtained
- by isolation of virus, which is enterovirus of type ECHO-7 *(Picomaviridae Enterovirus species Echovirus*), followed by
- adaptation of the virus to tumor tissue by serial passaging of it in tumor tissue, which is melanoblastoma from various patients, and selection of the strain having an affinity for tumor, whereby the adaptation of the virus to tumor tissue is performed according to the scheme of propagating the virus propagated in tumor tissue in test culture of human embryonic fibroblasts and again passaging the virus in tumor tissue, wherein on the whole 3-10 successive passages are carried out alternately in finite cultures from individual tumors and in test-cultures, and
- successive propagation of this adapted strain by passaging in human embryonic fibroblast culture up to a titer 5-7 log TCD 50 / 0.1 ml of the enterovirus of type ECHO-7, whereafter the propagated viral culture is isolated,
- the obtained enterovirus of type ECHO-7 being effective *in vivo* in suppression of tumor growth.

Enterovirus (family *Picomaviridae,* genus *Enterovirus,* species Echovirus) is known as one of the simplest RNA viruses. It has only 4 structural polypeptides and, accordingly, not as much antigenic determinants (active centers) for interactions with various clones of the immunocompetent cells.

The antitumoral effect of this immunostimulator affects mainly the humoral stage of the immune response (B-cells). It is known that in case of the induction of the malignant process the functional activity of B-cells is suppressed earlier than of T-cells.

The cluster of specific features of the method enables adaptation of the isolated nonpathogenic culture to tumor tissue of various patients as well as its propagation. In case if the titer of virus is lower than 5 log TCD 50/0.1ml the immunostimulating effect is not observed because the culture's activity is too weak.

If the titers are higher than 7 log TCD 5010.1ml the lowering of activity of the culture is observed.

The enteroviruses are recovered from the intestinal tract of men, mainly from healthy children 2-5 years of age and isolated in human embryonic primary culture. Then the nonpathogenic serotype *Picomaviridae Enterovirus* ECHO-7 are selected. The feces are used for the recovery of virus.

After the initial culture is produced its oncotropism for melanoblastoma or other tumors is detected in short-term finite cultures from tumors.

The selected culture is adapted by the serial passaging in tumor tissue of melanoblastoma from various patients to increase the specifity of the effect.

The passaged strain is propagated to the titer 5-7 log TCD 50/0.1ml in the culture of human embryonic fibroblasts.

The detailed description of the method is given below.

The initial nonpathogenic culture of *Picomaviridae Enterovirus* type ECHO-7, is recovered from the intestinal tract of healthy children 2-5 years of age. The fecal samples are used for the primary recovery of virus. The fecal sample in sterile flasks immediately are stored in refrigerator at -10°C. For the recovery of virus 20% suspension in Hankss' balanced salt solution is used. The suspension is centrifuged at 3000 rpm 30 min. The supernatant is freezed and stored at such temperature for 24 hours. Then the suspension is thawed, antibiotics are added: penicillin - 1000 U/ml, streptomycin - 250 U/ml and incubated 1-2 hours. The obtained suspension is centrifuged 1-2 times at 3000 rpm for 30 min. The final supernatant after testing for sterility is again freezed and stored at -10°C - -20°C.

With the produced virus-containing material the monolayer of trypsinized culture of human embryonic fibroblasts is inoculated. As the maintenance medium for inoculated cultures the Medium 199 with 2% of calf serum is used, as growth medium - Medium 199 with 10% calf serum.

After 1-hour contact with the cells the suspension is removed and maintenance medium is added. Then the cell culture tubes are incubated at 37°C for 2-10 days. During this period 2-3 serial passages of the material in human embryonic fibroblast cultures are performed. Incubation of tubes in any passage lasts till the complete degeneration of cells.

After isolation of cytopathogenic virus the produced material is titrated and serotyped.

The virus-containing material is centrifuged at 3000 rpm for 20 min, then freezed and stored at -20°C - -70°C.

The titer of virus in human embryonic fibroblast culture is 5-7 log TCD 50/0.1 ml.

After the isolation virus strains were tested for oncotropism in finite tumor cultures.

Adaptation of isolated virus culture is performed in tumor tissue obtained from operation material of various patients who had never undergone any treatment. The tumor tissue is purified from adipose tissue, necrotic lesions and blood. The purified tumor tissue is incubated for 18-20 hours at 0°C, then it is finely chopped in 0.1-0.2 mm pieces, imbedded in Medium 199 or Eagles medium without serum (100 mg tissue/4ml medium).

The tissue is infected with virus culture 10⁵-10⁶ TCD 50 and incubated in thermostat at 37°C without additional input of CO₂. 5ml fresh medium is changed daily. The cultivation is ended when the death of tumor pieces is achieved, what is checked morphologically and visually, according to the change of medium colour.

For the detection of the virus propagation intensity the titration of virus is performed in the liquid phase of tumor culture, which is sampled daily. The intensity of virus reproduction is evaluated by calculation of the percentage of the amount of virus detected in the infected culture to the infection dose. In case virus multiplication in tumor tissue is found, material from the days of maximum propagation is centrifuged at 3000 rpm for 20 min.

The adaptation of the culture is performed according to following scheme: the virus propagated in tumor tissue is passaged (propagated) in test-culture of human embryonic fibroblasts and again passaged in tumor tissue. On the whole 3-10 successive passages are carried out alternately in finite cultures from individual tumors and in test-cultures.

The initial virus strain is stored in frozen stage at -20°C - -70°C.

The propagation of the strain is performed in human embryonic fibroblast culture till the titer is 5-7 log TCD 50/0.1 ml with Medium 199 without serum and with addition of antibiotics: penicillin 100 U/ml, streptomycin 50 U/ml.

The indicator of pH - phenol red could be added into medium in concentration of 0.002%.

The liquid phase of the infected culture of human embryonic fibroblasts after the complete degeneration of cells is centrifuged at 3000 rpm for 20 min.

The produced immunostimulator with antitumoral effect is sterile liquid of rose colour. It is filled in vials by 2ml (2x10⁷ TCD 50) - it is the dose for one injection. The preparation is stored in frozen state.

The produced immunostimulator was applied for treatment of patients with malignant skin melanoma (T₁₋₃ N₀ M₀) after operative removal of either primary tumor or primary tumor together with regional lymph nodes.

The preparation is administered 2-4 weeks after operation, as parenteral (intramuscular) injection in upper lateral gluteal region.

The administration scheme is following: during the first three months one injection of 2 ml (2x10⁷ TCD 50 /day), 3 days in a row, after each course of treatment is an interval of three weeks, next three months - one injection/monthly. Then there is an interval of three months and monthly injections are repeated within 9th-12th postoperative months.

The administration of preparation could be commenced before the removal of primary tumor - 1-2 weeks prior the operation: one injection of 2 ml (2x10⁷ TCD 50 /day), 3 days in a row.

A longer period of administration of preparation is possible as well - up to 2-3 years of postoperative period with monthly injections of 2ml.

Administration of preparation causes practically no side effects.

Below are given the results of clinical trials of proposed immunostimulating preparation (ISVO - immunostimulator of viral origin), preparation of bacterial origin (C.P. - *Corinebacterium Parvum)* and a chemical preparation Decaris (Levamisole) for the malignant melanoma patients.

Decaris was administered according to the following scheme: tablets perorally twice a week with 3 days interval during 8 months from 2nd-4th week after removal of primary tumor.

Preparation C.P. was administered according to the following scheme: Immunostimulation was commenced 1-4 weeks after radical operation. During the first five days preparation was administered intravenously 1-4mg daily in ascending dosage. Then during a year preparation was administered subcutaneously once a week 1ml in 4 points in abdominal region.

The materials about the patients who were under the observation not less than 3 years were analysed.

Below, in Table 1 the data of survival and process dynamics in patients of skin melanoma after the radical operation are given.

**Table 1**

| Parameter obseved | Immunostimulator | | | Operation only |
|---|---|---|---|---|
| | C.P. | Decaris | ISVO | |
| Survival | 13:21 * | 22:28 | 21:25 | 54.5% |
| | 62% | 78.6% | 84% | |
| Stabilization of the process | 10:21 | 17:28 | 16:25 | 25.7% |
| | 47% | 60.7% | 64% | |

| | | | | |
|---|---|---|---|---|
| *- in the numerator is given the number of patients with positive findings vs total number of patients observed; in denominator- percentage of this ratio. | | | | |

The data demonstrate that the best results of survival were obtained in case of administration of proposed immunostimulator based on the enterovirus culture.

In Table 2 the data of mortality dynamics of melanoma patients after surgery and immunostimulation are given.

**Table 2**

| Group of patients observed | Number of patients who died during: | | |
|---|---|---|---|
| | the 1st year | 2 years | 3 years |
| C.P. | 1: 21 | 6: 21 | 8: 21 |
| | 5% | 29% | 38% |
| Decaris | 2: 28 | 6: 28 | 6: 28 |
| | 7% | 21% | 21% |
| ISVO | 0: 25 | 2: 25 | 4: 25 |
| | 0% | 8% | 16% |
| Operation only | | | 54.5% |

| | | | |
|---|---|---|---|
| * in the numerator is given the number of patients died vs total number of patients observed; in denominator -percentage of this ratio. | | | |

It could be concluded from the obtained data, that the immunostimulation provided by the proposed immunostimulator significantly increased survival of patients, since during the first year died nobody from the group treated with ISVO, whereas in other groups 5% and 7% died, respectively.

The difference in mortality is observed also after two years, in comparison with the second best preparation Decaris, ISVO induced 1.3 times better results.

The proposed preparation decreased the frequency of process progressing after the radical operation as well.

Below, in Table 3 the data on the frequency of process progressing in melanoma patients after radical operation and immunostimulation are given.

**Table 3**

| Period of time | Preparation | | | Operation only |
|---|---|---|---|---|
| | C.P. | Decaris | ISVO | |
| 3 years | 11: 21 | 11: 28 | 9:25 | 74.3% |
| | 53% | 39% | 36% | |

| | | | | |
|---|---|---|---|---|
| *- in the numerator is given the number of patients with process progressing vs total number of patients observed; in denominator- percentage of this ratio. | | | | |

As could be seen from the table, the slowest progression of the process was in case of treatment with the proposed preparation.

The pattern of dissemination of the tumor - skin melanoma after the radical operation in dependance from the preparation used is given in Table 4.

**Table 4**

| Immunostimulator | Relapses | Metastases | | | |
|---|---|---|---|---|---|
| | | Subcutaneous | Lymph nodes | Lungs | Brain |
| C.P. | 1:11 | 4: 11 | 6: 11 | 7: 11 | 3: 11 |
| | 9% | 36% | 55% | 63% | 27% |
| Decaris | no | 1: 8 | 4: 8 | 2: 8 | 1; 8 |
| | | 12.5% | 50% | 25% | 12.55 |
| ISVO | no | 3: 9 | 4: 9 | 1: 9 | 1: 9 |
| | | 33% | 45% | 11% | 11% |

| | | | | | |
|---|---|---|---|---|---|
| *- in the numerator is given the number of patients with relapses (metastases) vs total number of patients observed; in denominator - percentage of this ratio. | | | | | |

The data given in Table 4 indicate that in case of treatment with the proposed preparation the metastases are mostly subcutaneous and in lymph nodes, which could be detected and removed relatively easy. Metastases in the lungs are found 6 and 2.5 times more rarely than in case of treatment with another, known preparations.
The pattern of the progress of the disease among the patients who died is given in Table 5.

**Table 5**

| Group observed | Number of patients | Duration of stabilization (months) | Period of dissemination {monthsl | Duration of survival (months) |
|---|---|---|---|---|
| C.P. | 9 | 13 | 9 | 22 |
| Decaris | 6 | 10 | 5 | 15 |
| ISVO | 10 | 17 | 12 | 29 |

The data given in Table 6 indicate that the proposed preparation significantly extends the period of stabilization and period of dissemination, but duration of survival was prolonged for 12 months more than in case of Decaris and 7 months more than in case of preparation based on *Corinebacterium Parvum.*

Below in Table 6 the data of survival of gastric cancer patients after radical operation in the 3rd stage of the disease are given.

**Table 6**

| Period of observation | Survival of the patients (frequency in %) | |
|---|---|---|
| | Operation+ISVO | Operation |
| 2 years | 15: 21 * | 45.8 |
| | 71.41 | |
| 5 years | 10: 21 | 24.2 |
| | 47.6 | |

| | | |
|---|---|---|
| *-in the numerator is given the number of alive patients vs total number of patients observed; in denominator- percentage of this ratio. | | |

It could be concluded from the results presented, that in patients treated with proposed preparation frequency of survival is 1.5-2 times higher in comparison with operation alone.

Below in Table 7 the data of survival of patients of rectum cancer after radical operation are given.

**Table 7**

| Period of observation | Survival of the patients (frequency in %) | |
|---|---|---|
| | Operation+ISVO | Operation |
| 5 years | 10: 14 * | 58.2 |
| | 71.2 | |

| | | |
|---|---|---|
| *- in the numerator is given the number of alive patients vs total number of patients observed; in denominator - percentage of this ratio. | | |

As could be seen from the results, administration of the proposed preparation increased survival for 14%.

Below are given the results of immune status control during the treatment with the proposed immunostimulator (ISVO).

### 1 . Reaction of DTH to the tumor-associated melanoma antigen

There was no significant changes in the frequency of positive DTH reaction in patients treated with ISVO during the first 6 months (50-60%). After resuming immunostimulation (9-12 months) this index was elevated in 10% of patients, what means that frequency of normal reactions of DTH are not changing or increasing during the stimulation with ISVO.

In case of treatment with Decaris the frequency of normal reactions of DTH undergo changes, there is no reaction in 35% of patients after 2 months. In part of patients the frequency of this reaction undergoes significant changes (20-30%).

As the reaction of DTH displays the ability of the organism to distinguish, react and reject the tumor-associated antigen, the stabilization or elevation of this index are favourable indications, but sharp fluctuations and lowering of this reaction are unfavourable indications of the immune status in course of immunostimulation. Thus, according to this index, ISVO performs better than Decaris.

### 2. RILA for tumor-associated melanoma antigen

The treatment with ISVO in postoperative period does not cause sharp fluctuations of reactions. The frequency of detection of this reaction in the course of immunostimulation has a tendency to increase. Among the patients treated with Decaris the frequency of detection of this reaction in the course of stimulation lowered from 80% to 30%, what means, that by the end of the course leucocytes of only 30% patients recognized and reacted with melanoma antigen. RILA as well as DTHR displays the ability of T-lymphocytes to distinguish and react with tumor-associated antigen. The abscence or lowering of this reactivity should be regarded as tumor resistance weakening.

It should be mentioned, that after the treatment with Decaris was ceased, this index returns to the initial value.

### 3. The inhibitor of adherence of leucocytes in serum

During the course of immunostimulation with ISVO the frequency of detection of this index rises from 35% at the beginning of the course to 80% at the end. In the group of patients treated with Decaris the frequency of detection of this index lowers from 40% in the beginning of the course to 20% in the end of stimulation course.

The inhibitor of adherence of leucocytes detected in RILA is detected in 60 - 80% of healthy people. The same index is found in melanoma patients after radical operation and ISVO treatment, and at the stabilization stage. The inhibitor dissappears during the dissemination process, what means, that presence of the inhibitor of adherence of leucocytes in serum is one of the indices of normal immune status. The appearance of it in 45% of patients treated with ISVO is regarded as the positive effect of immunostimulation.

In comparison, in the Decaris treated group after 4 months of treatment the inhibitor is found only in 20% of patients.

### 4. The blocking factor in serum, detected in RILA

The blocking factor displays the state of blockade of immunocompetent cells participating in the processes of antitumoral immunity.

This factor is detected in 25-30% patients after the primary melanoma lesion has been removed. In the patients treated with ISVO, in the 6-th month of immunostimulation this factor is detected only in 10% of patients.

The blocking factor in serum is also lowered during the first 2 months of stimulation with Decaris and is detected in 10% of patients, but on the 6-th month the blocking factor is detected in 30-40% of patients, including such patients, who did not present the blocking factor after removal of the tumor.

### 5. Antiviral antibodies in serum

During the first months of immunostimulation with ISVO there is a sharp increase of antiviral antibodies in serum, which are detected by reaction of neutralization and reaction of inhibition of hemagglutination. During this period the titers of antiviral antibodies increase from 1:20 to 1:5000-10000. After immunostimulation is terminated (6-9 months) titers of antibodies are falling and then become stable with resumption of immunostimulation (9-12 months).

It indicates that an active viral immunogenesis takes place in patients' organisms. The period of viral immunogenesis is favourable for the expression of antitumoral immunity via recurrence and normalization of processes, blocked by the malignant growth.

It means that administration of ISVO in postoperative period after radical removal of primary melanoma lesion, either makes stable the immune reactions of the organism or activates them.

Besides, no signs either of hyperstimulation or of autoimmune reactions were observed. The indices of the immune status present evidence that the toxic effects were not observed during lasting parenteral administration of ISVO.

The proposed immunostimulator based on nonpathogenic, tumor-adapted enterovirus strain of type ECHO *Picomaviridae Enterovirus* is effective in suppression of tumor growth.

This type of virus belongs to unenveloped viruses and because of that does not carry antigens of cellular origin, which could induce autoimmune reactions.

Moreover, characteristic of this type of virus is the genetic safety, based on organization of the genome, which does not integrate into host cell genome and does not recombinate with the genomes of other viruses.

The method developed for producing of the immunostimulator ensures the maximum activity of the preparation obtained.

The proposed immunostimulator is actively suppressing malignant growth in case of relapses, extending the period of stabilization and time of dissemination. Moreover, metastases predominantly are subcutaneous and in lymph nodes, which could be removed comparatively easy.

## Claims

1. A method for preparation of an immunostimulator with antitumoral effect comprising non-pathogenic virus strain, **characterized in that** this strain is obtained
- by isolation of virus, which is enterovirus of type ECHO-7 *(Picornaviridae Enterovirus species Echovirus),* followed by
- adaptation of the virus to tumour tissue by serial passaging of it in tumour tissue, which is melanoblastoma from various patients, and selection of the strain having an affinity for tumour, whereby the adaptation of the virus to tumour tissue is performed according to the scheme of propagating the virus propagated in tumour tissue in test culture of human embryonic fibroblasts and again passaging the virus in tumour tissue, wherein on the whole 3-10 successive passages are carried out alternately in finite cultures from individual tumours and in test-cultures, and
- successive propagation of this adapted strain by passaging in human embryonic fibroblast culture up to a titre 5-7 log TCID50 / 0.1 ml of the enterovirus of type ECHO-7, whereafter the propagated viral culture is isolated,
- the obtained enterovirus of type ECHO-7 being effective *in vivo* in suppression of tumour growth.

2. The method according to claim 1, **characterized in that** the said enterovirus strain has been isolated from the intestinal tract of 2-5 years old children.

3. A non-pathogenic enterovirus of type ECHO-7 *(Picornaviridae Enterovirus species Echovirus)* obtained by the method of claims 1 or 2.

## Patentansprüche

1. Verfahren zur Herstellung eines Immunstimulators mit anti-tumoralem Effekt umfassend einen nicht-pathogenen Virusstamm, **dadurch gekennzeichnet, dass** dieser Stamm erhalten wird
- durch Isolation eines Virus, welches ein Enterovirus des Typs ECHO-7 (*Picornaviridae Entervirus species Echovirus)* ist, gefolgt durch
- Adaption des Virus an Tumorgewebe durch dessen serielles Passagieren in Tumorgewebe, welches Melanoblastom von verschiedenen Patienten ist, und Selektion des Stammes mit Tumoraffinität, wobei die Adaption des Virus an Tumorgewebe nach dem Virusvermehrungsschema durchgeführt wird wie in Tumorgewebe in Testkultur humaner embryonaler Fibroblasten vermehrt und abermaligem Passagieren des Virus in Tumorgewebe, wobei insgesamt 3-10 sukzessive Passagen alternierend in finiten Kulturen von individuellen Tumoren und in Test-kulturen durchgeführt werden, und
- sukzessives Vermehren dieses adaptierten Stammes durch Passagieren in humanen embryonalen Fibroblastenkulturen bis zu einem Titer von 5-7 log TCID50/0,1 ml des Enterovirus des Typs ECHO-7, wonach die vermehrte Viruskultur isoliert wird,
- der erhaltene Enterovirus des Typs ECHO-7 *in vivo* effektiv in der Suppression von Tumorwachstum ist.

2. Verfahren nach Anspruch 1, **dadurch gekennzeichnet, dass** besagter Enterovirusstamm aus dem Darmtrakt 2-5 Jahre alter Kinder isoliert worden ist.

3. Nicht-pathogener Enterovirus des Typs ECHO-7 *(Picornaviridae Entervirus species Echovirus),* erhalten nach dem Verfahren nach den Ansprüchen 1 oder 2.

## Revendications

1. Procédé de préparation d'un immunostimulateur avec un effet antitumoral comprenant une souche de virus non pathogène, **caractérisé en ce que** cette souche est obtenue
- par isolement de virus, qui est un entérovirus de type ECHO-7 (*Picornaviridae Enterovirus espèce Echovirus),* suivi de
- adaptation du virus à un tissu tumoral par des passages en série de celui-ci dans le tissu tumoral, qui est un mélanoblastome provenant de divers patients, et la sélection de la souche présentant une affinité pour la tumeur, moyennant quoi l'adaptation du virus au tissu tumoral est effectuée selon le schéma de propagation du virus propagé dans le tissu tumoral dans une culture à tester de fibroblastes embryonnaires humains et à nouveau passage du virus dans le tissu tumoral, dans lequel globalement 3 à 10 passages successifs sont réalisés en alternance dans des cultures finies provenant de tumeurs individuelles et dans des cultures à tester, et
- la propagation successive de cette souche adaptée par passage dans une culture de fibroblastes embryonnaires humains jusqu'à un titre de 5 à 7 TCID50/0,1 ml de l'entérovirus de type ECHO-7, après quoi la culture virale propagée est isolée,
- l'entérovirus de type ECHO-7 obtenu étant efficace *in vivo* dans la suppression de la croissance tumorale.

2. Procédé selon la revendication 1, **caractérisé en ce que** ladite souche d'entérovirus a été isolée à partir du système intestinal d'enfants âgés de 2 à 5 ans.

3. Entérovirus de type ECHO-7 non pathogène *(Picornaviridae Enterovirus espèce Echovirus)* obtenu par le procédé selon les revendications 1 ou 2.
